# EUROPEAN PATENT APPLICATION

(11) **EP 0 824 022 A1**
(43) Date of publication of application: **18.02.1998**
(21) Application number: 96112925.1
(22) Date of filing: 12.08.1996
(51) Int. Cl.: A61M 5/168

(54) **Parenteral drug administration system**

(71) Applicant: Microflow Engineering SA, 2007 Neuchâtel (CH)
(72) Inventor: Hess, Joseph, 2022 Bevaix (CH)
(74) Representative: Patry, Didier Marcel Pierre

(57) **Abstract**

System for the parenteral administration of a drug to a patient, comprising :
- a supply (2) of liquid including an active drug component and a non-active component,
- means (3, 4, 5, 6) for parenterally supplying said liquid to said patient, and
- means (10) for temporarily introducing an additional quantity of said drug into drug supply means comprising :
   - a drug supply passage (11) having an inlet and an outlet,
   - a drug reservoir (12) containing said drug and in fluid communication with said inlet,
   - drug propulsion means (13) for producing a pressure pulse in the drug in said drug supply passage (11) to cause a predetermined amount of drug to be displaced towards and ejected from said outlet in the form of a microdroplet, and
   - control means (16) for selectively operating said drug propulsion means (13).

## Description

The present invention relates generally to drug administration systems, and in particular to systems for the administration of a therapeutic drug to a patient by parenteral, or non-respiratorial, means. The invention is applicable in the medical field for the controlled delivery of drugs, for example for pain management, by intravenous delivery. It will be convenient to hereinafter describe the invention in relation to such an application, although it should be appreciated that the invention is not limited to that application.

Various systems for the administration of a drug by parenteral means are known. One type of commonly used system comprises a bag containing a homogeneous mixture of an active drug to be administered to the patient and a non-active liquid, such as a hydrating solution. This bag is suspended beside a patient's bed by a support frame. A flexible tube is provided between the outlet of the bag an the inlet of a dripping chamber. The outlet of this latter is connected via a tube to an infusion set placed in the patient's arm. An adjustable clamp surrounds the tube interconnecting the bag and the dripping chamber. By appropriately constricting the tube with the clamp, the rate at which the liquid mixture in the bag drips into the dripping chamber, and thus the rate at which the active drug is delivered to the patient, can be controlled.

Whilst such a system has proven to be adequate in many circumstances, this system is nevertheless ill-suited to applications which require a temporary increase in the rate at which the therapeutic drug is administered to the patient. An example of such an application is that of Patient Controlled Analgesics, or PCA, in which a patient is provided with means to temporarily increase the flow rate of a pain relieving drug delivered to him.

The above-described system is relatively inexpensive, but lacks the precision and means of control required in PCA applications so that the patient does not over-administer the drug to himself.

In order to address this problem, some parenteral drug administration systems use more sophisticated means of controlling the flow of liquid in the bag delivered to the patient. It is known to use, for example, a syringe pump whereby a mechanical motor drives the plunger of a syringe filled with liquid. The liquid flow rate is controlled by the speed at which the motor drives the syringe plunger.

However, such systems are also relatively expensive and, due to mechanical imprecisions in the motor/syringe arrangement, do not provide a suitably accurate drug delivery in many cases. Moreover, poorly controlled increases in the total flow rate and pressure of the liquid delivered to the patient can lead to local swelling and fluid retention at the point of drug delivery in the patient and even to vein rupture. In addition, such systems are inflexible and cumbersome, and are not well adapted for use in ambulatory or implantable applications.

It is an object of the present invention to provide a parenteral drug administration system which alleviates or overcomes the problems of existing parenteral drug administration systems.

Another object of the invention is to provide a parenteral drug administration system which is accurate, and easily adaptable to a variety of applications and inexpensive.

A further object of the invention is to provide a parenteral drug administration system which is well-suited for use in applications which require a temporary increase in the rate at which the therapeutic drug is administered to the patient, such as PCA applications.

With this in mind, the present invention provides a system for the parenteral administration of a drug to a patient, comprising a supply of liquid including an active drug component and a non-active component, and means for parenterally supplying said liquid to said patient, characterised in that it further comprises means for temporarily introducing an additional quantity of said drug into drug supply means comprising a drug supply passage having an inlet and an outlet, a drug reservoir containing said drug and in fluid communication with said inlet, drug propulsion means for producing a pressure pulse in the drug in said drug supply passage to cause a predetermined amount of drug to be displaced towards and ejected from said outlet in the form of a microdroplet, and control means for selectively operating said drug propulsion means.

This arrangement provides for a temporarily increase in the concentration of the drug by means of a supplementary source of that drug, and in such a way that there results only a minimal increase in the overall total flow rate of liquid delivered to the patient. The additional drug is introduced into the liquid delivered to the patient by a combination of features which provides for an extremely accurate, low-cost parenteral drug delivery which is particularly suitable for applications in which a temporary increase in the rate at which the therapeutic drug is administered to the patient.

The following description refers in more detail to the various features of the parenteral drug administration system of the present invention. To facilitate an understanding of the invention, reference is made in the description to the accompanying drawings where different embodiments of the parenteral drug administration system are illustrated. It is to be understood that the parenteral drug administration system of the present invention is not limited to the embodiments as illustrated in the drawings.

In the drawings :
- Figure 1 is a schematic block diagram of one embodiment of a parenteral drug administration system according to the invention;
- Figure 2 is a schematic view of one embodiment of an active unit dose device forming part of the parenteral drug administration system shown in figure 1;
- Figure 3 is a schematic view of part of the active unit dose device of figure 2;
- Figure 4 is a schematic view of a variation of the part of the active unit dose device of figure 3;
- Figure 5 is a side view of a flow sensor forming part of the parenteral drug administration system shown in figure 1;
- Figure 6 is a plan view of a flow sensor forming part of the parenteral drug administration system shown in figure 1;
- Figure 7 is a schematic view of a chip section for use in the control unit forming part of the parenteral drug administration system shown in figure 1;
- Figure 8 is a graphical representation of the output signals of the chip section shown in figure 7 and the corresponding frequency of the voltage pulses applied to the active unit dose device of figure 2;
- Figure 9 is a schematic diagram of a CMOS chip for use in the control unit forming part of the parenteral drug administration system shown in figure 1.

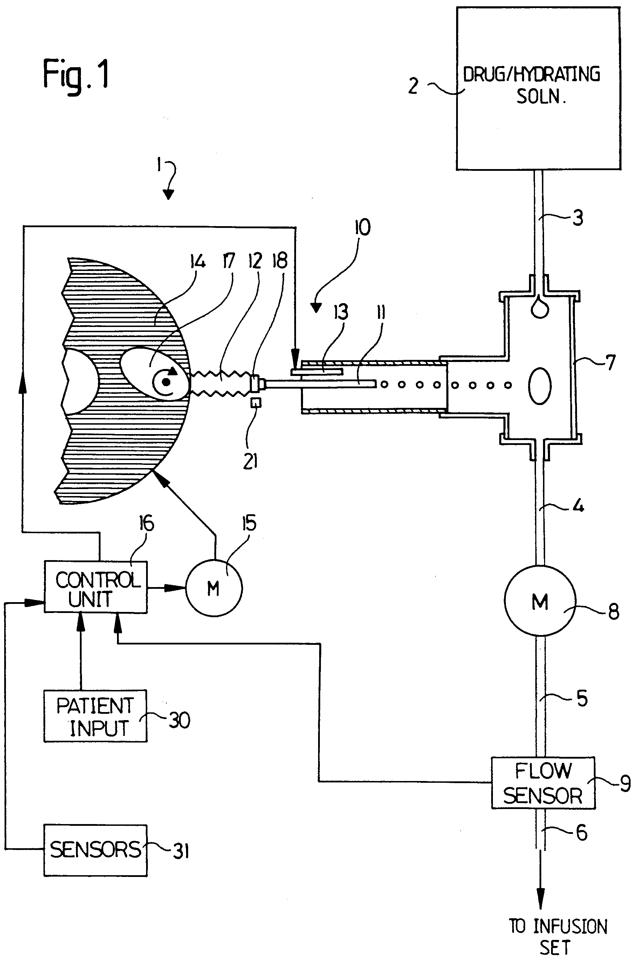

Referring now to figure 1, there is shown a schematic representation of one embodiment of the parenteral drug administration system according to the invention.

The parenteral drug administration device 1 comprises a bag 2 containing a supply of liquid including an active drug component, such as morphine or other pain relieving therapeutic drug, and a non-active component, such as a hydrating solution. The liquid contained in the bag 2 is parenteraly supplied to a patient by means of tubes 3, 4, 5 and 6, which interconnect a mixing piece 7, a pump 8 and a flow sensor 9. In one application, the bag 2 may be suspended from a support frame, located, for example, next to a patient requiring the administration of the active drug, so that the liquid contained in the bag 2 is forced therefrom by gravity. In other embodiments, the bag 2 and the other components comprising the drug administration system of the present invention may be arranged in a portable or implantable device and the liquid contained in the bag 2 may be forced therefrom by capillary action, the application of pressure to the bag 2, or other appropriate means.

In normal operation, the outlet of the tube 6 is connected to an infusion set placed, for example, intravenously into a patient. The liquid in the bag 2 is provided under gravity to the pump 8 which is set so as to provide the liquid at a predetermined flow rate to the patient. Preferably, the pump 8 is constituted by a low coast peristaltic pump which can easily be used in conjunction with the other features of the drug administration system 1. The pump 8 may be required, for example, in gravity-fed systems, where it is necessary to introduce the drug-bearing solution to the patient at a defined pressure. It will be appreciated however, that in applications not presenting this requirement, the pump 8 may be omitted from the arrangement shown in figure 1.

In addition, the parenteral drug administration system 1 includes means 10 for introducing an additional quantity of drug into the liquid provided to the patient. The drug introducing means 10 comprise a drug supply passage 11, a drug reservoir 12 containing the additional drug to be provided to the patient, and drug proportion means 13. The drug contained in the reservoir 12 is provided to the inlet of the drug supply passage 11. The drug is supplied to the interior of the drug supply passage 11 under pressure or by means of capillary action. The drug propulsion means 13 act to produce a pressure pulse on the drug in the drug supply passage 11 to cause a predetermined amount of the drug to be displaced towards and ejected from the outlet of the drug supply passage 11 in the form of a microdroplet. These microdroplets are supplied to one of the inputs of the mixing piece 7 when the drug propulsion means are operated. One or more drug reservoirs, which may each contain a unit-dose such as 30 to 50 microlitres or other suitable small amount of drug insulation or suspension, may be radially supported on or in a magazine 14 so that, upon rotation of the magazine 14 by a motor 15, each reservoir 12 may be placed, in turn, in a delivery position as shown by the placement of the drug reservoir 12 in figure 1. The magazine may be caused to rotate in this manner by an electronic control circuit 16 which controls the operation of a motor 15.

In synchronisation with the rotational movement of the magazine 14, a reservoir pressurising device 17 is provided in this embodiment which compresses the collapsible reservoir 12 so as to provide the drug contained in the reservoir 12 to the interior of the drug supply passage 11. The reservoir pressurising device 17 is preferably provided by a spring loaded eccentric cam located at a fixed position under the collapsible reservoir. Conveniently, the drug supply passage 11 may be constituted by a tube made of glass or like material such as ceramic or alumina having an outlet of defined diameter and may be mounted to the drug reservoir by a holder 18.

The drug propulsion means 13 may be constituted by one or more electroresistive elements which are mounted to the outer surface of the glass tube constituting the drug supply passage 11, and in particular may be constituted by piezoelectric devices. The electronic control unit 16 is connected to the electroresistive elements 13 and is adapted to provide them with a series of voltage pulses. When, under the control of the electronic control circuit 16, one of these voltage pulses is applied to one of the electroresistive elements 13, that element undergoes a deformation. This deformation is transmitted to a portion of the drug supply passage 11, whereby a pressure pulse is applied to the drug therein. This pressure pulse drives the drug in the drug supply passage 11 downstream from the electroresistive elements 13 towards the outlet of the drug supply passage 11 and ejects the drug therefrom in the form of a microdroplet.

The drug supply passage 11, the drug reservoir 12 and the drug propulsion means 13 may conveniently manufactured as a single, preferably disposable unit. These units may be mounted in a disposable magazine, which by being moved to the correct position allows each such active unit dose device to deliver its dose on request. In such a way, a high precision device is provided which may conveniently be manufactured separately at a low cost and easily incorporated into existing parenteral drug administration systems.

The magazine 14 can be an injection moulded wheel that carries a number of such active unit dose devices, appropriately held in a position therearound, or the magazine can be formed to carry a blister tape, the blister forming the collapsible reservoirs on which the tube and piezoelectric actuator are appropriately attached.

It will also be appreciated that there exists a variety of actuations or motor possibilities to move a disposable magazine containing a number of the said disposable active unit dose devices in a rotational way such as to present one disposable active unit device after the other in front of the input to the mixing piece 7 for actuation and drug delivery thereto.

In another embodiment, particularly suited as an added device to gravity-feed type administration systems, the disposable active unit dose device can be implemented as a cylinder or pen-shaped, unit dose rechargeable drug delivery device, which may contain no or only a few additional spare disposable active unit dose devices and will have a snap-on type positioning mechanism.

In one embodiment, each active unit dose device uses a tube 11 made substantially of alumina due to its excellent frequency matching with piezoelectric devices and its wides produced as a substrate for printing circuits.

Extruded, annular piezoelectric actuators suitable for use in the invention may be formed in a number of ways. However, in a preferred embodiment, the piezoelectric actuators are deposited on the surface of the injected tube by a widely used spring printed method, in order to reduce costs and increase the operation of flexibility of operation of the resulting device. This fabrication method, using ink containing PZT (lead zirconate titinate) material, as well as the ensuing firing results in a mass production processes leading to low cost production. This fabrication method also allows the deposition of one or more piezoelectric actuators, each constituting of the actuator PZT material and electrodes made of a suitable metal on the outer surface of the tube 11 and/or on another part of the active unit dose device in a way to produce appropriately size microdroplets and at a frequency suitable for controlled drug delivery. In that sense, the active unit dose device represents a low cost drug delivery pump suitable as an add-on device to parenteral systems where the microdroplets are delivered into the main liquid delivery system.

In another embodiment, the tube 11 is at least partially produced by ceramic injection moulding, a process which provides a high quality smooth and chemically amorphous interior surface as well as very low production coast in large quantities. The moulding process also allows for shaping of the tube interior for optimal flow and microdroplets production.

The disposable character of such an active unit dose device allows for a cost effective design of the key components of the parenteral drug administration device of the present invention.

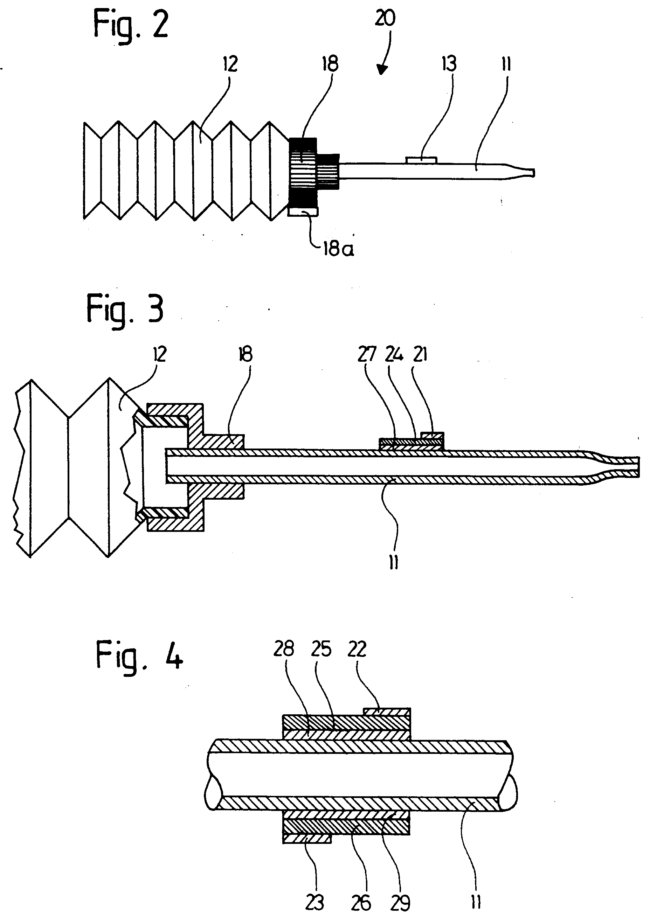

Figure 2 shows a side view of such an active unit dose device. The active unit dose device 20 comprises the tube 11, drug reservoir 12, electroresistive device 13 and holder 18 shown in figure 1.

It should be noted that for the purpose of delivering drug solutions as well as suspensions, the tube holder 18 may be required to have certain properties. For drug delivery as a solution, the tube holder 18, like the reservoir 12 itself, is preferably an injection moulded part made of a suitable medical grade polymer. For suspensions, however, the holder 18 may be made as a ceramic injection moulded component, allowing a piezoelectric actuator frequency in effect to function as a resonance stirring agent. Preferably, an additional piezoelectric actuator 18b acting as such a resonant stirring agent, and operating at the same or a different frequency to that of the electroresistive device 13, is fixed to the holder or to the reservoir 12. Other medical grade injection moulded materials, such as 316L stainless steel, may also be used for the purpose of the invention.

In a case of emulsions, for example oil-in-water emulsions, a short heating pulse may be required to be applied to the drug to be administrated in some cases for viscosity control. To this effect, a heating resistor 21, has shown in figure 1 may be mounted on the tube holder 18 or elsewhere on the active unit dose device. In this case, the holder 18 is preferably made of an injection moulder ceramic material. Connections to the heating resistor may be made via spring loaded connecting elements or other suitable connection means.

Figures 3 and 4 show to embodiments of the active unit dose device in which one or more such heating elements or resistors 21, 22, 23 are mounted on corresponding electrodes 24, 25, 26. These latter are themselves mounted on PZT actuators 27, 28, 29 as previously described.

Referring again to figure 1, the parenteral drug administration system 1 comprises patient operable input means 30, such as a simple push-button for example, for providing an input signal to the control unit 16. The control unit 16 may be adapted to selectively operate the piezoelectric devices 13 in response to the operation of the patient operable input means 30. In this way, a patient or physician is able, by causing the operation of the piezoelectric device 13, to enable the drug contained in the reservoir 12 to be delivered into the flow of liquid ordinarily supplied to the patient from the bag 2, thereby temporarily increasing the concentration of the drug in the liquid supplied to the patient.

The control circuitry 16 may be programmed or otherwise adapted so that the delivery of the on demand dose from the active unit dose device and the corresponding increase of concentration of the drug in the liquid supplied to the patient depends upon a number of factors such as the total dosage, the time of dosage, allowable on-demand dose, the lock-out time between operations, the evaluation of direct or indirect diagnostic signals and patient control functions.

In that regard, the drug administration system 1 of figure 1 may also comprise one or more sensors 31 to detect relevant biophysiological data to modify the dosage supplied from the active unit dose device in response to this data.

In another embodiment, the control circuitry 16 may also be provided with biochronological programming so as to modify this dosage. For example, the dosage may be delivered at predetermined times during the biological or circadian rhythm reason of a patient, depending upon need.

One advantage of the drug administration device of the present invention is that the piezoelectric device 13 does not participate in the normal continuous drug delivery from the bag via the peristaltic motor or other appropriate device to the patient. The active unit dose device is only actuated on demand, with suitable lock-out times as previously described to deliver small amounts of drug only. The infrequent operation of the piezoelectric device 13 results in this part of the drug administration system having a long lifetime with respect to the lifetime of piezoelectric actuators considered for continuous use, for example in implantable drug delivery pumps.

In order to further enhance its precision, the parenteral drug administration device 1 may comprise a flow sensor 9. Preferably, this flow sensor 9 is constituted by a piezoelectric differential pressure sensor. Since the present invention does not rely on significantly increased total flow rates during the temporary increase in the supply of an active drug to a patient, but rather on minute injections of additional drug solution into the administration set, for example 5,000 microliters over 30 minutes or over an hour, a flow sensor with a resolution of preferably not less than one microliter per hour may conveniently be used.

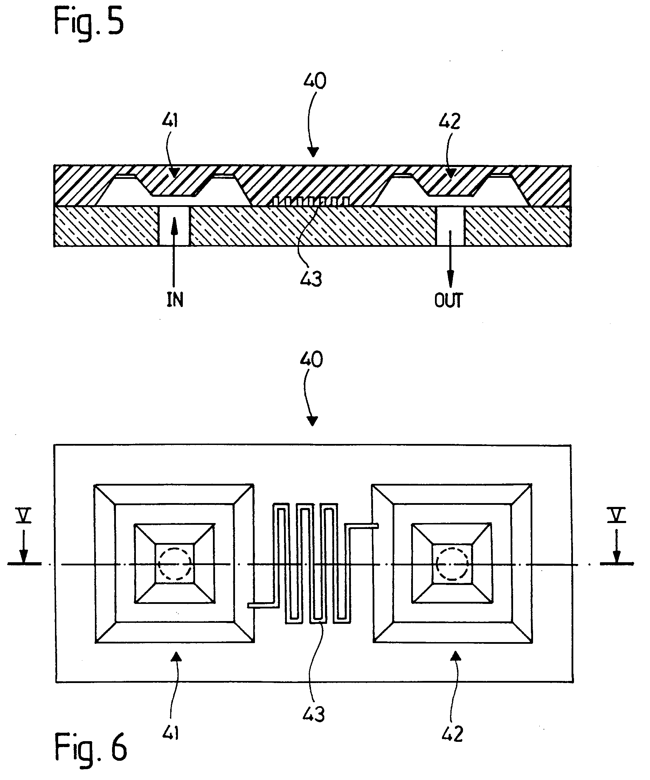

This may be achieved by using a micromachined differential pressure sensor 40, as shown in figures 5 and 6. The flow sensor 40 comprises two piezoelectric low pressure sensors 41 and 42 interconnected by a microchannel 43 through which the liquid delivered to the patient flown. For a known viscosity, that is to say for a known drug-bearing liquid, the pressure drop across the microchannel between the two pressure sensors corresponds to the volumetric flow of that drug. A signal from each pressure sensor is supplied from the flow sensor 9 to the control unit 16.

In addition, an integrated temperature sensor (not shown) may be included on the differential pressure sensor chip in order to allow for compensation of viscosity or other changes due to temperature variation if needed. Various ranges of flow rates can be achieved by modifying the cross-section and/or the length of the microchannel 43. Flow rates from 500 microliters per hour to 20 milliliters per hour have been achieved by the applicant with a precision of one percent full scale, and up to 100 microliters per hour can be achieved with a flow sensor of this type. The micromachined channel is preferably made from an amorphous ceramic substrate, its construction thus making it suitable for purging, sterilisation and reuse. In order to avoid entry of other particles, a medical grade filter may be mounted upstream from the flow sensor. In conjunction with the electronic circuitry 16, the above-described micromachined differential pressure and temperature sensors enable the measurement and control of flow rate, volume, infusion pressure, occlusion and other controllable parameters, so that the active unit dose device is able to provide precise drug concentration control and recording of on-demand increases in drug concentration supplied to the patient.

The control unit 16 preferably comprises one or more fuzzy logic circuit elements. The feature of fuzzy logic circuitry is that uncertain linguistic information can be handled qualitatively using membership functions. Fuzzy logic circuitry is thus ideally suited to the diagnosis of medical conditions derived, for example, from the biophysiological data detected by the sensors 31. Advantageously, such circuitry may be realized without the complexity, high calculating power and high power consumption necessitated by classical digital and analog circuitry, thus reducing the cost and power consumption of the drug delivery system according to the invention, as well as enhancing its portability.

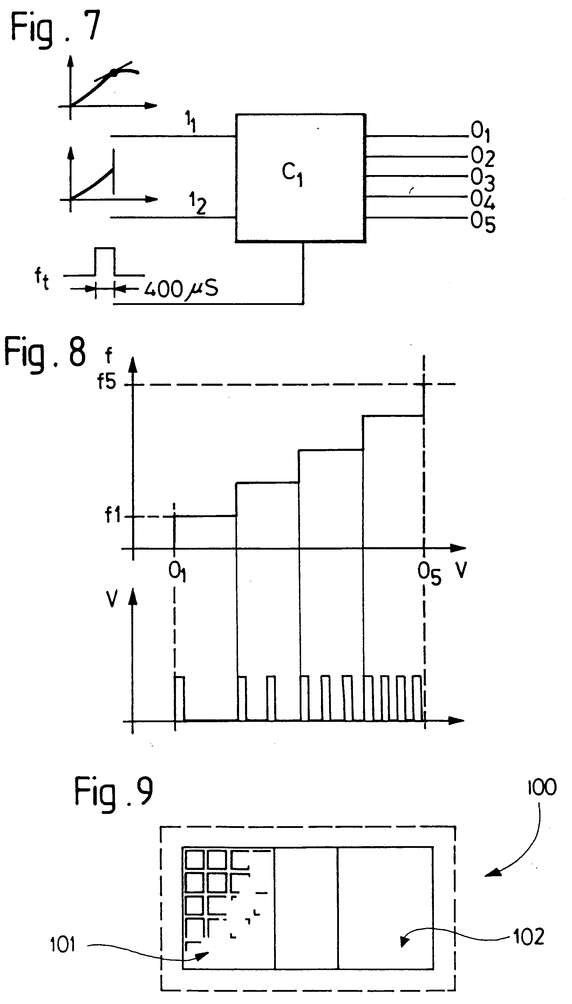

In a preferred embodiment of the invention shown in figures 7 to 9, the control unit 16 of figure 1 may be realized by a CMOS chip 100 which comprises two sections, one section 101 containing a very low power analog CMOS fuzzy rule processing module for reservoir compression and position control, and for diagnostic sensor interface via curve mirroring. The CMOS chip 100 also comprises a section 102 realized in a high voltage low current CMOS (for example 40 to 80 volts) for piezoelectric actuation with frequency curve mirroring according to diagnostic sensor inputs or fixed demand and frequency operations according to the desired temporal increase in drug concentration and other drive function rule processing.

The very low power analog CMOS fuzzy rule chip section 101 of the ship 100 consists of a number of analog CMOS rule circuits C1 which for example each have two inputs and five more outputs. Such a chip-section C1 can contain for example seventy such rule circuits C1 on a one millimeter square chip-surface. It processes the input signals from the sensor 31 in time slices of, for example, 400 microseconds, over any time duration. The inputs I1, I2 mirror the output signal from the sensors 31 by one input receiving the sensor output signal directly and the other input by measuring the slope or change of that output signal completed with respect to the last reading which was taken, for example 400 microseconds before, if that was the cycle time. As shown in figure 8, the outputs 01 to 05 follow a set of rules allowing to adjust the actuation frequency and/or the amplitude, for example, in five steps each, of the piezoelectric actuator, in order to mirror the diagnostic sensor curve so that a precise dose each of the higher concentration drug in the drug reservoir 12 is delivered into the infusion set, in the form of a string of microdroplets, each microdroplet having the same spherical shape and volume as determined by the dimension of the active unit dose device.

It can be appreciated that such a solution enables to initiate and stop drug delivery at any point and at any time, since the actuation of the piezoelectric device 13 can be electronically stopped after a given number of microdroplets have been ejected from the tube 11. It is therefore possible to vary the concentration of the drug in the fluid applied to the patient in a manner which is physiologically responsive to the patient needs.

Those skilled in the art will appreciate that many variations and modifications may be made to the configuration described hereabove with departing from the ambit of the present invention.

In that regard, it will be appreciated that the present invention is applicable to virtually all integral and intravenous drug administration systems, whether gravity-fed or pump fed, and whether they are bed-side, ambulatory, disposable - for example elastomeric or osmotic -, implantable systems, key-hole surgery, endoscopic devices or catheter applications.

For example, when the system is to be used as an implantable device, the patient operable input means may comprise a radio receiver appropriately implanted into the patient body, for detecting a radio diffused control signal emitted from a patient operable transmitter. Moreover, the bag 2 may be replaced by an implantable liquid supply. The reservoir 12 and the implantable liquid supply may be replenished respectively with the active drug and the active drug bearing liquid by means of implantable ports which provide access to the interior thereof via the exterior of the patient.

The above described drug administration system provides not only the ability to deliver an on-demand dose for drug concentration increase but also the ability to progressively increase the concentration over time of the drug delivered from the reservoir into the main liquid flow path by following an appropriate diagnostic sensor curve. In addition, the ability to initiate the active unit dose device at any time or point of the sensor input signal response results in the drug administration system being independent from the main liquid supply means used in the drug administration system, be it gravity or pump feed, bag, cassette or syringe. The active dose unit device is therefore able to be produced independently of and is readily adaptable for use with a wide variety of drug delivery systems.

## Claims

1. System for the parenteral administration of a drug to a patient, comprising :
- a supply (2) of liquid including an active drug component and a non-active component, and
- means (3, 4, 5, 6) for parenterally supplying said liquid to said patient,
characterised in that it further comprises
- means (10) for temporarily introducing an additional quantity of said drug into drug supply means comprising :
- a drug supply passage (11) having an inlet and an outlet,
- a drug reservoir (12) containing said drug and in fluid communication with said inlet,
- drug propulsion means (13) for producing a pressure pulse in the drug in said drug supply passage (11) to cause a predetermined amount of drug to be displaced towards and ejected from said outlet in the form of a microdroplet, and
- control means (16) for selectively operating said drug propulsion means (13).

2. System according to claim 1, characterised in that it further comprises
- pressurizing means (17) for pressurizing the drug in said drug reservoir (12).

3. System according to claim 2, characterised in that said drug reservoir (12) is constituted by a collapsible recipient, and in that said pressurizing means (17) comprise an actuator for bearing against said recipient.

4. System according to any one of the preceding claims, characterised, in that said drug supply passage (11) is constituted by a tube mounted to said drug reservoir (12) by a holder (18).

5. System according to claim 4, characterised in that said tube (11) is substantially made of glass, alumina, ceramics or stainless steel.

6. System according to any one of the preceding claims, characterised in that said drug propulsion means (13) comprise at least one piezoelectric device deposited on the outer surface of said drug supply passage (11).

7. System according to claim 6, characterised in that said drug propulsion means (13) further comprise at least one layer (27; 28, 29) containing PZT material.

8. System according to any one of the preceding claims, characterised in that said drug supply passage (11), said drug reservoir (12) and said drug propulsion means (13) at least partly form a single-use active unit dose device.

9. System according to claim 8, characterised in that it further includes at least one heating element (21; 22, 23) mounted on said active unit dose device.

10. System according to any one of the preceding claims, characterised in that it further comprises a piezoelectric actuator (18b) acting as a resonant stirring agent which is fixed to the drug reservoir (12) or to the holder (18).

11. System according to any one of the preceding claims, characterised in that it further comprises patient operable input means (30), and in that said control means (16) is adapted to selectively operate said drug propulsion means (13) in response to the operation of said patient operable input means (30).

12. System according to any one of the preceding claims, characterised in that it further comprises at least one sensor (31) for detecting biophysical data from said patient, and in that said control means (16) is adapted to selectively operate said drug propulsion means (13) in response to said biophysical data.

13. System according to any one of the preceding claims, characterised in that it further comprises a piezoelectric differential flow sensor (9) for measuring the total flow of said liquid supplied to said patient and for providing a feedback signal to said control means (16) representative of said total flow.
